(19) Europäisches Patentamt / European Patent Office / Office européen des brevets

(11) **EP 3 025 144 B1**

(12) **FASCICULE DE BREVET EUROPEEN**

(45) Date de publication et mention
de la délivrance du brevet:
**24.05.2017 Bulletin 2017/21**

(21) Numéro de dépôt: **14747543.8**

(22) Date de dépôt: **21.07.2014**

(51) Int Cl.:
*G01N 21/45* (2006.01)    *G01N 21/47* (2006.01)
*G01N 33/483* (2006.01)    *G03H 1/00* (2006.01)
*G03H 1/04* (2006.01)    *G01N 15/14* (2006.01)
*C12M 1/00* (2006.01)    *G01N 21/03* (2006.01)
*G01N 21/85* (2006.01)    *G01N 15/10* (2006.01)

(86) Numéro de dépôt international:
**PCT/EP2014/065636**

(87) Numéro de publication internationale:
**WO 2015/011096 (29.01.2015 Gazette 2015/04)**

(54) **PROCÉDÉ POUR TRIER DES CELLULES ET DISPOSITIF ASSOCIÉ**

ZELLSORTIERVERFAHREN UND -VORRICHTUNG

CELL-SORTING METHOD AND ASSOCIATED DEVICE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **23.07.2013 FR 1357240**

(43) Date de publication de la demande:
**01.06.2016 Bulletin 2016/22**

(60) Demande divisionnaire:
**17165937.8**

(73) Titulaire: **Commissariat à l'Énergie Atomique et aux Énergies Alternatives 75015 Paris (FR)**

(72) Inventeur: **ALLIER, Cédric 38000 Grenoble (FR)**

(74) Mandataire: **Brevalex 95, rue d'Amsterdam 75378 Paris Cedex 8 (FR)**

(56) Documents cités:
**WO-A1-2012/062805    US-B1- 6 905 838**

• **JAMES P. RYLE ET AL: "Multispectral lensless digital in-line holographic microscope: LED illumination", PROCEEDINGS OF SPIE, vol. 7717, 30 avril 2010 (2010-04-30), pages 77170P-77170P-8, XP055095750, ISSN: 0277-786X, DOI: 10.1117/12.854960**
• **SU TING-WEI ET AL: "High-throughput lensfree imaging and characterization of a heterogeneous cell solution on a chip", BIOTECHNOLOGY AND BIOENGINEERING, WILEY & SONS, HOBOKEN, NJ, US, vol. 102, no. 3, 15 février 2009 (2009-02-15), pages 856-868, XP002584870, ISSN: 0006-3592, DOI: 10.1002//BIT.22116 [extrait le 2008-09-08]**

**Description**

**DOMAINE TECHNIQUE**

La présente invention concerne le domaine des procédés pour discriminer une cellule vivante d'une cellule morte.

L'invention concerne également un dispositif pour mettre en oeuvre un tel procédé.

**ÉTAT DE LA TECHNIQUE ANTÉRIEURE**

On connaît dans l'art antérieur différents procédés pour discriminer une cellule vivante d'une cellule morte, ou en d'autres termes une cellule viable d'une cellule non-viable.

On connaît par exemple des procédés de microscopie à fluorescence. Ces procédés utilisent un microscope optique pour visualiser un échantillon, ainsi qu'un marqueur fluorescent qui se fixe préférentiellement sur une catégorie de cellules, parmi les deux catégories cellules vivantes et cellules mortes. Le marqueur fluorescent absorbe un rayonnement incident à une première longueur d'onde, et émet en réponse un rayonnement de fluorescence à une deuxième longueur d'onde. On nomme « cellules marquées » les cellules fixées au marqueur fluorescent. On voit qu'en éclairant un échantillon de cellules marquées avec un rayonnement à la première longueur d'onde, on peut discriminer une cellule vivante d'une cellule morte en identifiant les cellules émettant le rayonnement de fluorescence à la deuxième longueur d'onde. En pratique, on va observer au microscope des cellules présentant une couleur correspondant à la longueur d'onde du rayonnement de fluorescence, et des cellules ne présentant pas cette couleur.

Pour marquer les cellules vivantes, on peut par exemple utiliser la calcéine AM en tant que marqueur fluorescent se fixant uniquement aux cellules vivantes. On connaît également l'iodure de proprium se fixant uniquement aux cellules mortes.

Un inconvénient de ces procédés est qu'ils sont invasifs : l'échantillon de cellules est perturbé par l'ajout du marqueur fluorescent.

Un autre inconvénient de ces procédés est qu'ils sont onéreux : le microscope à fluorescence doit présenter d'excellentes performances optiques basées sur l'utilisation de composants optiques de grande qualité. De plus, leur champ est limité, souvent inférieur à 1 mm$^2$, ce qui ne permet pas l'observation simultanée d'un grand nombre de cellules.

On connaît aussi le brevet US 6,905,838, qui enseigne de discriminer une cellule vivante d'une cellule morte grâce à une image de la cellule obtenue par illumination en champ sombre.

On connaît également la demande WO 2012/062805, selon laquelle une cellule agit comme une lentille, et peut donc être caractérisée par son plan focal et le pic d'intensité lumineuse sur ce plan focal.

On connaît également l'article de Ting-Wei Su & al, "High-throughput lensfree imaging and characterization of a heterogeneous cell solution on a chip", Biotechnology and bioengineering, Vol 102, No. 3, February 15, 2009, qui enseigne de façon générale l'utilisation d'un système d'imagerie sans lentille pour décompter et caractériser les particules d'un échantillon.

Un objectif de la présente invention est de proposer un procédé pour discriminer une cellule vivante d'une cellule morte, qui ne présente pas au moins l'un des inconvénients de l'art antérieur.

En particulier, un but de la présente invention est de proposer un procédé économique, convenant particulièrement à l'observation simultanée d'un grand nombre de cellules, par exemple plusieurs centaines voire plusieurs milliers.

On souhaite également proposer un procédé non invasif, qui ne nécessite pas l'ajout d'une substance de marquage.

**EXPOSÉ DE L'INVENTION**

La présente invention est définie par un procédé pour discriminer une cellule vivante d'une cellule morte, mis en oeuvre à l'aide d'un dispositif d'imagerie sans lentille comprenant une source lumineuse et un photodétecteur matriciel, dans lequel :

- on illumine, à l'aide de la source lumineuse, un échantillon d'une solution liquide comprenant des cellules ; et
- on acquiert, à l'aide du photodétecteur matriciel disposé en face de la source lumineuse, une figure de diffraction globale de l'échantillon, la figure de diffraction globale comprenant une pluralité de figures de diffraction élémentaires associées chacune à une cellule, chaque figure de diffraction élémentaire présentant une succession de courbes fermées concentriques.

Selon l'invention, on classe une cellule dans l'une parmi les deux catégories cellule vivante ou cellule morte, en fonction d'une intensité lumineuse sur une surface centrale d'une figure de diffraction élémentaire associée à ladite cellule.

De préférence, ladite surface centrale est inscrite dans une surface délimitée par un plus petit anneau de diffraction de la figure de diffraction élémentaire.

Le procédé selon l'invention peut comprendre les étapes suivantes :

- on détermine une valeur d'un indicateur numérique représentatif de l'intensité lumineuse dans ladite surface centrale ;
- on compare la valeur de l'indicateur numérique à une valeur de seuil prédéterminée ; et
- en fonction du résultat de cette comparaison, on classe ladite cellule dans l'une parmi les deux catégories cellule vivante ou cellule morte.

Avantageusement, l'indicateur numérique est une intensité maximale ou une intensité moyenne ou une valeur crête-à-crête d'intensité.

La valeur de seuil prédéterminée est de préférence une intensité de référence, et :

- une valeur de l'indicateur numérique inférieure à ladite intensité de référence correspond à une cellule vivante ; et
- une valeur de l'indicateur numérique supérieure à ladite intensité de référence correspond à une cellule morte.

Le procédé selon l'invention est avantageusement mis en oeuvre pour effectuer un suivi *in situ* d'un traitement cellulaire dans un bioréacteur.

On peut illuminer l'échantillon à l'aide d'une source lumineuse spatialement cohérente.

Avantageusement, on acquiert la figure de diffraction globale de l'échantillon, à l'aide d'un photodétecteur matriciel disposé en face de la source lumineuse, aucun dispositif de grossissement n'étant disposé entre l'échantillon et ledit photodétecteur matriciel.

L'invention concerne également un dispositif pour discriminer une cellule vivante d'une cellule morte comprenant :

- une source lumineuse, agencée pour illuminer un échantillon d'une solution liquide comprenant des cellules ; et
- un photodétecteur matriciel disposé en face de la source lumineuse, formant avec la source lumineuse un dispositif d'imagerie sans lentille, et agencé pour acquérir une figure de diffraction globale de l'échantillon, la figure de diffraction globale comprenant plusieurs figures de diffraction élémentaires associées chacune à une cellule, chaque figure de diffraction élémentaire présentant une succession de courbes fermées concentriques.

Selon l'invention, le dispositif comprend des moyens de calcul :

- recevant en entrée la figure de diffraction globale ;
- déterminant l'intensité lumineuse sur une surface centrale d'une figure de diffraction élémentaire associée à une cellule ; et
- fournissant en sortie un classement de ladite cellule dans l'une parmi les deux catégories cellule vivante ou cellule morte.

De préférence, aucun dispositif de grossissement n'est disposé entre l'échantillon et le photodétecteur matriciel.

Le dispositif selon l'invention peut comprendre :

- un premier logement étanche, recevant la source lumineuse et présentant une première fenêtre transparente entre la source lumineuse et le photodétecteur matriciel ;
- un second logement étanche, recevant le photodétecteur matriciel, et présentant une seconde fenêtre transparente entre la première fenêtre transparente et le photodétecteur matriciel ;

la distance entre la première fenêtre transparente et la seconde fenêtre transparente étant variable, le premier logement étanche, la source lumineuse, le second logement étanche, et le photodétecteur matriciel formant ensemble une sonde immergeable.

Selon un premier mode de réalisation de l'invention, la distance entre la première fenêtre transparente et la seconde fenêtre transparente est variable dans le temps, la première fenêtre transparente et la deuxième fenêtre transparente définissant deux plans parallèles entre eux, et le dispositif selon l'invention comprenant des moyens pour déplacer la seconde fenêtre transparente relativement à la première fenêtre transparente.

Les moyens pour déplacer la seconde fenêtre transparente relativement à la première fenêtre transparente peuvent comprendre une crémaillère entraînée en translation par un pignon, et fixe relativement au second logement étanche.

Selon un deuxième mode de réalisation de l'invention, la distance entre la première fenêtre transparente et la seconde fenêtre transparente est variable dans l'espace.

La première fenêtre transparente et la deuxième fenêtre transparente peuvent définir deux plans inclinés l'un relativement à l'autre, de sorte que la distance entre la première fenêtre transparente et la seconde fenêtre transparente dépend de l'emplacement sur la seconde fenêtre transparente.

Avantageusement, la source lumineuse est spatialement cohérente.

Les moyens de calcul peuvent déterminer l'intensité lumineuse sur une surface centrale d'une figure de diffraction élémentaire associée à une cellule, la surface centrale étant inscrite dans une surface délimitée par un plus petit anneau de diffraction de la figure de diffraction élémentaire.

BRÈVE DESCRIPTION DES DESSINS

La présente invention sera mieux comprise à la lecture de la description d'exemples de réalisation donnés à titre purement indicatif et nullement limitatif, en faisant référence aux dessins annexés parmi lesquels :

- la Figure 1 illustre un dispositif connu d'imagerie sans lentille ;
- les Figures 2A et 2B illustrent une figure de diffraction obtenue à l'aide du dispositif de la Figure 1 et pour une cellule vivante ;

- les Figures 3A et 3B illustrent une figure de diffraction obtenue à l'aide du dispositif de la Figure 1 et pour une cellule morte ;
- la Figure 4A illustre un disque central de la figure de diffraction représentée en Figure 2A ;
- la Figure 4B illustre un disque central de la figure de diffraction représentée en Figure 3A ;
- les Figures 4C et 4D illustrent un indicateur numérique selon l'invention ;
- la Figure 5 illustre l'évolution de la figure de diffraction associée à une cellule, lorsque ladite cellule initialement vivante est progressivement rendue non viable jusqu'à ce qu'elle meurt ;
- la Figure 6 illustre de façon schématique un dispositif pour mettre en oeuvre le procédé selon l'invention ;
- la Figure 7 illustre de façon schématique un premier mode de réalisation de sonde immergeable selon l'invention ;
- la Figure 8 illustre de façon schématique un deuxième mode de réalisation de sonde immergeable selon l'invention ; et
- la Figure 9 illustre de façon schématique un bioréacteur selon l'invention.

## EXPOSÉ DÉTAILLÉ DE MODES DE RÉALISATION PARTICULIERS

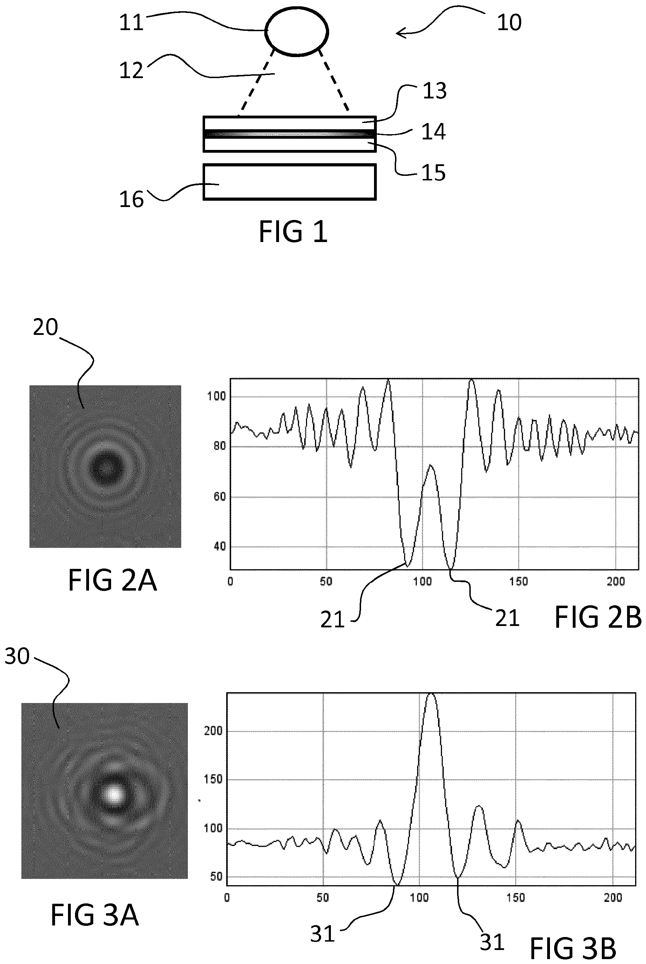

On va tout d'abord décrire, en référence à la Figure 1, un dispositif d'imagerie sans lentille 10. Un tel dispositif est connu de l'homme de l'art, qui saura facilement trouver dans la littérature les détails de réalisation d'un tel dispositif.

Une source lumineuse 11 émet un faisceau lumineux 12 illuminant un échantillon 14. La source lumineuse est, de préférence, cohérente spatialement. La source lumineuse peut être une diode laser, ou une diode électroluminescente (LED) suivie d'un trou de filtrage. Le trou de filtrage permet d'améliorer la cohérence spatiale du faisceau lumineux émis par la LED. Avantageusement, la source lumineuse est également temporellement cohérente.

On utilise par exemple une LED dont le spectre d'émission est centré sur 450 nm, soit une émission dans le bleu. La largeur du pic à 450 nm est de 40 nm. La puissance d'émission de la LED est par exemple comprise entre 100 mW et 1 W. La LED est suivie par un trou de filtrage de diamètre 150 $\mu$m, placé directement au contact de la LED.

L'échantillon 14 est placé entre la source lumineuse 11 et un photodétecteur matriciel 16.

Dans l'exemple représenté sur la Figure 1, l'échantillon 14 est un échantillon liquide placé entre deux lamelles 13, 15 transparentes à la longueur d'onde d'émission de la source lumineuse. Le dispositif 10 selon l'invention travaille donc en transmission. La distance entre la lamelle 13 (du côté de la source lumineuse) et la source lumineuse est généralement comprise entre 1 cm et 10 cm.

L'échantillon 14 est par exemple un échantillon d'un liquide biologique tel que le sang, une solution de culture de cellules, un échantillon prélevé dans la nature tel qu'un échantillon de l'eau d'un lac, etc. Le dispositif 10 permet de détecter des particules contenues dans l'échantillon et présentant un faible diamètre, typiquement entre 100 nm et 500 $\mu$m.

Le photodétecteur matriciel 16 convertit un rayonnement électromagnétique incident en un signal électrique analogique. Ce photodétecteur matriciel 16 est généralement relié à un convertisseur analogique-numérique de façon à fournir une image numérique. On parle de photodétecteur matriciel, car la surface de détection du photodétecteur est découpée en pixels formant une matrice. Le photodétecteur matriciel 16 est par exemple un capteur CCD (pour l'anglais « *Charge-Coupled Device* ») ou un capteur CMOS (pour l'anglais «*Complementary Metal Oxide Semiconductor* »). Chaque pixel du photodétecteur est par exemple un carré de côté inférieur à 9 $\mu$m, et même inférieur à 5 $\mu$m, par exemple 2,2 $\mu$m. En particulier, on peut utiliser un capteur CMOS de 24 mm$^2$, présentant un pas de pixel de 2,2 $\mu$m.

Le photodétecteur matriciel 16 détecte une figure de diffraction correspondant à l'influence des particules de l'échantillon 14 sur le faisceau lumineux 12. En particulier, le photodétecteur matriciel 16 détecte une figure de diffraction, correspondant aux interférences entre une onde lumineuse incidente provenant directement de la source lumineuse 11 et une onde lumineuse diffractée par des particules de l'échantillon 14. On nomme « hologramme » une telle figure de diffraction. Un avantage d'une telle figure de diffraction est que le signal détecté est de grande amplitude, grâce à la contribution du signal provenant directement de la source lumineuse. Un autre avantage de cette figure de diffraction est que le champ de vision détecté est large, par exemple supérieur à 20 mm$^2$. Le photodétecteur 16 est positionné à proximité de l'échantillon 14, par exemple à 0,8 mm de la lamelle 15 (lamelle du côté du photodétecteur 16).

On détecte donc une figure de diffraction correspondant à un objet, et non directement l'image de cet objet. On nomme cette technique « imagerie sans lentille ». Dans tout le texte, on nomme « dispositif d'imagerie sans lentille » un dispositif de ce type, pour détecter une figure de diffraction correspondant à un objet. Il faut noter qu'un tel dispositif peut comprendre une matrice de microlentilles, servant à focaliser sur chaque pixel le faisceau correspondant aux interférences à détecter. Cependant, un tel dispositif ne comporte pas d'optique de grossissement disposée entre l'objet et le photodétecteur.

L'homme du métier saura aisément positionner les uns par rapport aux autres chacun des éléments parmi la source lumineuse 11, l'échantillon 14 et le photodétecteur 16.

On pourra prévoir un support (non représenté) pour recevoir l'échantillon 14, ce support étant disposé entre la source lumineuse 11 et le photodétecteur 16.

Selon l'invention, on utilise ce dispositif 10 avec un échantillon 14 d'une solution liquide comprenant des cellules. Par exemple, on utilise une solution de cellules cancéreuses HeLa. On choisit en particulier d'utiliser un échantillon volumique : l'épaisseur entre les deux lamelles 13, 15 est par exemple de 0,2 mm. Le volume d'échantillon 14, imagé sur le photodétecteur 16 sous la forme d'une figure de diffraction, est d'environ 5 $\mu$L.

On acquiert ainsi, sur le photodétecteur 16, une figure de diffraction globale correspondant aux interférences entre des ondes provenant directement de la source lumineuse 11 et des ondes émises par la source lumineuse puis diffractées par les cellules de l'échantillon 14. La figure de diffraction globale comprend plusieurs figures de diffraction élémentaires. Chaque figure de diffraction élémentaire est associée à une cellule de l'échantillon 14, et correspond aux interférences entre des ondes provenant directement de la source lumineuse 11 et des ondes émises par la source lumineuse puis diffractées par ladite cellule.

On a remarqué qu'une figure de diffraction élémentaire présente un profil caractéristique selon que la cellule est vivante ou morte.

La Figure 2A illustre une figure de diffraction élémentaire 20 associée à une cellule vivante HeLa. La figure de diffraction élémentaire 20 est obtenue à l'aide du dispositif décrit ci-dessus. Sur la Figure 2A, l'axe des abscisses et l'axe des ordonnées forment ensemble le plan du photodétecteur 16. Les teintes foncées correspondent à des zones de faible intensité lumineuse, et les teintes claires correspondent à des zones de forte intensité lumineuse. On observe sur la Figure 2A une alternance d'anneaux sombres et clairs nommés « anneaux de diffraction ». Ces anneaux de diffraction traduisent le profil circulaire des cellules observées. La figure de diffraction élémentaire 20 présente une symétrie axiale autour d'un axe de symétrie passant par le centre commun des anneaux sombres et clairs.

La Figure 2B illustre une coupe de cette figure de diffraction élémentaire 20, dans un plan passant par ledit axe de symétrie. Sur la Figure 2B, l'axe des abscisses correspond à une distance, graduée en pixels. L'axe des ordonnées correspond à une intensité lumineuse, graduée en niveau de gris (de 0 à 255, soient 256 niveaux de gris pour un signal analogique en sortie du photodétecteur 16 converti en signal numérique codé sur 8 bits). Une faible valeur du niveau de gris correspond à une faible intensité lumineuse. Une forte valeur du niveau de gris correspond à une forte intensité lumineuse. On reconnaît sur la Figure 2B l'axe de symétrie de la figure de diffraction élémentaire 20, cet axe étant défini par une droite d'abscisse 105 pixels. Autour de cet axe, on distingue un pic central présentant un maximum dont le niveau de gris vaut environ 70, entouré de deux pics secondaires dont les niveaux de gris valent environ 110. Ce pic central de faible intensité lumineuse se retrouve sur la Figure 2A, sur laquelle on voit que les anneaux sont centrés sur un disque gris.

La Figure 3A illustre une figure de diffraction élémentaire 30 associée à une cellule morte HeLa. La figure de diffraction élémentaire est obtenue à l'aide du dispositif décrit ci-dessus. Là-encore, on observe une alternance d'anneaux sombres et clairs. La figure de diffraction élémentaire 30 présente une symétrie axiale autour d'un axe de symétrie passant par le centre commun des anneaux sombres et clairs.

La Figure 3B illustre une coupe de cette figure de diffraction élémentaire 30, dans un plan passant par ledit axe de symétrie.

Les Figures 3A et 3B correspondent respectivement aux Figures 2A et 2B, mais cette fois-ci dans le cas d'une cellule morte.

On observe sur la Figure 3B l'axe de symétrie de la figure de diffraction élémentaire 30, cet axe étant défini par une droite d'abscisse 105 pixels. Autour de cet axe, on distingue un pic central dont le niveau de gris vaut environ 250, entouré de deux pics secondaires dont les niveaux de gris valent environ 110. Ce pic central de forte intensité lumineuse se retrouve sur la Figure 3A, sur laquelle on voit que les anneaux sont centrés sur un disque blanc.

La différence entre la figure de diffraction élémentaire 20 associée à une cellule vivante et la figure de diffraction élémentaire 30 associée à une cellule morte est suffisamment significative pour permettre une détection de l'état de la cellule à partir de sa figure de diffraction. On va donc pouvoir exploiter cette différence pour discriminer une cellule vivante d'une cellule morte.

On remarque que les deux figures de diffraction élémentaires diffèrent en particulier sur une zone centrale, ici en forme de disque, définie sur une figure de diffraction. Ce disque est centré sur le centre des anneaux de ladite figure de diffraction élémentaire. Il est délimité par le premier anneau d'une figure de diffraction élémentaire. Ce premier anneau correspond ici à l'anneau d'intensité lumineuse minimale. On reconnaît sur la Figure 2B, respectivement 3B, les minima d'intensité 21, respectivement 31, correspondant à ce premier anneau de la figure de diffraction élémentaire. Le disque central est par exemple un disque de moins de 100 pixels de diamètre.

On peut relever qu'une modification du profil d'une cellule, par exemple pour prendre une forme ovale, se traduira par une modification de la figure de diffraction élémentaire associée. La figure de diffraction élémentaire associée présentera toujours une succession de courbes fermées concentriques, que par abus de langage on nommera également « anneaux de diffraction ». Ces courbes fermées entoureront une surface délimitée par un plus petit anneau de diffraction élémentaire.

L'idée à la base de l'invention consiste à utiliser une intensité lumineuse sur une surface centrale d'une figure de diffraction élémentaire, pour classer une cellule correspondante dans la catégorie cellule vivante ou dans la catégorie cellule morte. Cette surface est dite centrale, car elle est concentrique avec les anneaux de diffraction. La surface centrale est notamment inscrite dans une surface délimitée par un plus petit anneau de diffraction. Une telle surface correspond par exemple au disque central tel que défini ci-avant.

On voit donc que l'invention offre un procédé pour discriminer une cellule vivante d'une cellule morte, qui ne nécessite pas l'ajout d'une substance de marquage.

Les moyens matériels pour mettre en oeuvre ce procédé sont simples et peu onéreux : il s'agit en particulier d'un dispositif d'imagerie sans lentille. Le dispositif d'imagerie ne nécessite pas l'utilisation d'optiques coûteuses. L'invention ne nécessite pas l'utilisation de moyens de calcul très puissants, car on ne cherche pas à reconstruire une image des cellules à partir de leurs figures de diffraction élémentaires. On ne cherche pas non plus à traiter des figures de diffraction élémentaires pour les comparer à une bibliothèque de figures de diffraction de référence. On classe une cellule simplement en utilisant une intensité lumineuse sur une surface prédéterminée.

En outre, puisque l'on ne cherche pas ici à reconstruire l'image d'une cellule, il n'est pas nécessaire de connaître la distance entre la cellule et le photodétecteur. On peut donc facilement analyser une population de cellules contenues dans un volume, et pas seulement dans un plan.

Il n'est pas non plus nécessaire de prévoir une focalisation sur un plan particulier, ce qui permet là-encore d'analyser facilement une population de cellules contenues dans un volume.

Un autre avantage du procédé selon l'invention est sa précision : on peut réaliser un comptage précis d'un nombre de cellules vivantes et d'un nombre de cellules mortes, dans une région donnée.

On va maintenant illustrer plus précisément l'utilisation d'une intensité lumineuse sur une surface centrale d'une figure de diffraction élémentaire.

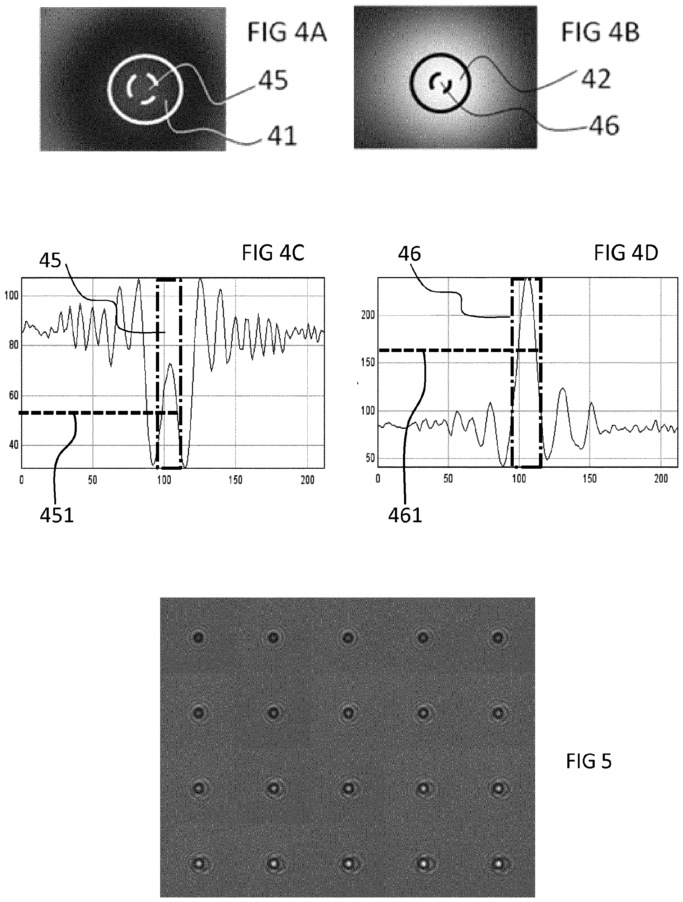

La Figure 4A illustre le disque central 41 tel que défini ci-avant, de la figure de diffraction représentée en Figure 2A. On voit que ce disque central est foncé. Il correspond à de faibles intensités lumineuses, ce qui est caractéristique d'une cellule vivante. Le disque central 41 présente ici un diamètre total d'environ 30 pixels. On peut définir sur le disque central 41 une surface centrale 45, ici un disque concentrique de diamètre inférieur ou égal à celui du disque central 41.

La Figure 4B illustre le disque central 42 tel que défini ci-avant, de la figure de diffraction représentée en Figure 3A. On voit que ce disque central est clair. Il correspond à de fortes intensités lumineuses, ce qui est caractéristique d'une cellule morte. On peut définir sur le disque central 42 une surface centrale 46, ici un disque concentrique de diamètre inférieur ou égal à celui du disque central 42.

On voit donc qu'il suffit d'évaluer l'intensité lumineuse sur une surface centrale inscrite dans le disque central 41 ou 42, pour savoir si une cellule correspondante est vivante ou morte.

On pourra définir un indicateur numérique, servant à caractériser l'intensité lumineuse dans la surface centrale 45 ou 46. Dans l'exemple représenté sur les Figures 4C et 4D, cet indicateur numérique est la valeur moyenne du niveau de gris dans la surface centrale. Puisque la figure de diffraction élémentaire présente ici une symétrie axiale, on peut travailler sur une coupe de cette figure de diffraction passant par l'axe de symétrie. La Figure 4C correspond à la Figure 2B, sur laquelle on a identifié une surface centrale 45 selon l'invention. L'indicateur numérique est identifié à la Figure 4C par la droite 451 d'ordonnée 55.

La Figure 4D correspond à la Figure 3B, sur laquelle on a identifié une surface centrale 46 selon l'invention. L'indicateur numérique est identifié à la Figure 4D par la droite d'ordonnée 160.

Selon l'invention, on utilise la valeur de cet indicateur numérique pour classer une cellule dans la catégorie cellule vivante ou cellule morte.

On fixe une valeur de seuil prédéterminée, séparant des valeurs d'indicateur numérique associées à une cellule vivante et des valeurs d'indicateur numérique associées à une cellule morte.

Dans le cas présent, on prévoit plus particulièrement une intensité de référence :

- un indicateur numérique inférieur à cette intensité de référence correspond à une cellule vivante ; et
- un indicateur numérique supérieur à cette intensité de référence correspond à une cellule morte.

L'intensité de référence, exprimée en niveau de gris, est par exemple fixée entre 80 et 150, typiquement 140.

Ainsi, il suffit, pour distinguer une cellule vivante d'une cellule morte, de :

- calculer ledit indicateur numérique ; puis
- comparer l'indicateur numérique à une valeur seuil prédéterminée.

La valeur seuil peut notamment être établie expérimentalement.

On pourra prévoir différentes types d'indicateurs numériques, sans sortir du cadre de la présente invention. Par exemple, on pourra considérer un maximum absolu d'intensité lumineuse, une amplitude crête à crête de l'intensité lumineuse, etc.

On a pris ici l'exemple des cellules HeLa, mais on pourra ainsi classer tout type de cellule.

Dans l'exemple représenté sur les figures, une cellule vivante correspond à une faible intensité lumineuse au centre de la figure de diffraction élémentaire, alors qu'une cellule morte correspond à une forte intensité lumineuse au centre de la figure de diffraction élémentaire. Le profil caractéristique de la figure de diffraction élémentaire associée à une cellule vivante ou une cellule morte peut dépendre du type de cellule étudié. Ainsi, il pourra arriver qu'une faible intensité lumineuse au centre de la figure de diffraction élémentaire corresponde à une cellule morte et qu'une forte intensité lumineuse au centre de la figure de diffraction élémentaire corresponde à une cellule vivante.

La surface centrale selon l'invention peut être une surface de dimensions prédéterminées, par exemple un disque centré sur le centre des anneaux de diffractions de la figure de diffraction élémentaire, et de diamètre prédéterminé. Pour fixer ce diamètre prédéterminé, on peut suivre les étapes suivantes :

- on considère qu'une cellule a pour figure de diffraction une tâche d'Airy. Le diamètre du plus petit anneau sombre de la figure de diffraction élémentaire est défini par la distance entre la cellule et le photodétecteur, et par un angle θ tel que $\sin \theta = 1,22\frac{\lambda}{d}$, où λ est la longueur d'onde d'émission de la source lumineuse, et d est le diamètre d'une cellule. Le diamètre du plus petit anneau sombre définit le diamètre du disque central 41, 42. Connaissant l'épaisseur de l'échantillon, la longueur d'onde de la source lumineuse et un ordre de grandeur du diamètre des cellules, on peut évaluer un intervalle de valeurs du diamètre du disque central 41, 42.
- on sélectionne la valeur inférieure de cet intervalle ;
- on définit le diamètre prédéterminé de la surface centrale comme étant inférieur ou égal à cette valeur inférieure d'intervalle.

En variante, on pourra identifier sur chaque figure de diffraction élémentaire le diamètre du plus petit anneau sombre. Le diamètre de la surface centrale est par exemple égal à une fraction fixe du diamètre du plus petit anneau sombre.

Le diamètre du disque central est compris par exemple entre 10 et 50 pixels (même entre 20 et 50 pixels). Le diamètre de la surface centrale est par exemple compris entre 5 et 50 pixels (même entre 10 et 50 pixels).

La Figure 5 illustre l'évolution de la figure de diffraction élémentaire associée à une cellule, lorsque ladite cellule initialement vivante est progressivement détériorée jusqu'à ce qu'elle meurt. La cellule HeLa, initialement vivante, est soumise à une température passant de 37°C à 45°C en dix minutes. On a relevé toutes les 30 secondes une figure de diffraction élémentaire de la cellule. Les figures de diffraction élémentaires sont rangées dans l'ordre chronologique de leurs acquisitions, de gauche à droite et de bas en haut. On identifie bien une évolution progressive de la figure de diffraction élémentaire depuis une figure telle que représentée en Figure 2A vers une figure telle que représentée en Figure 3A. Cette évolution est monotone : le pic central devient progressivement de plus en plus lumineux. La Figure 5 confirme bien que l'on peut définir un seuil d'intensité sur une surface centrale selon l'invention, pour discriminer une cellule vivante d'une cellule morte.

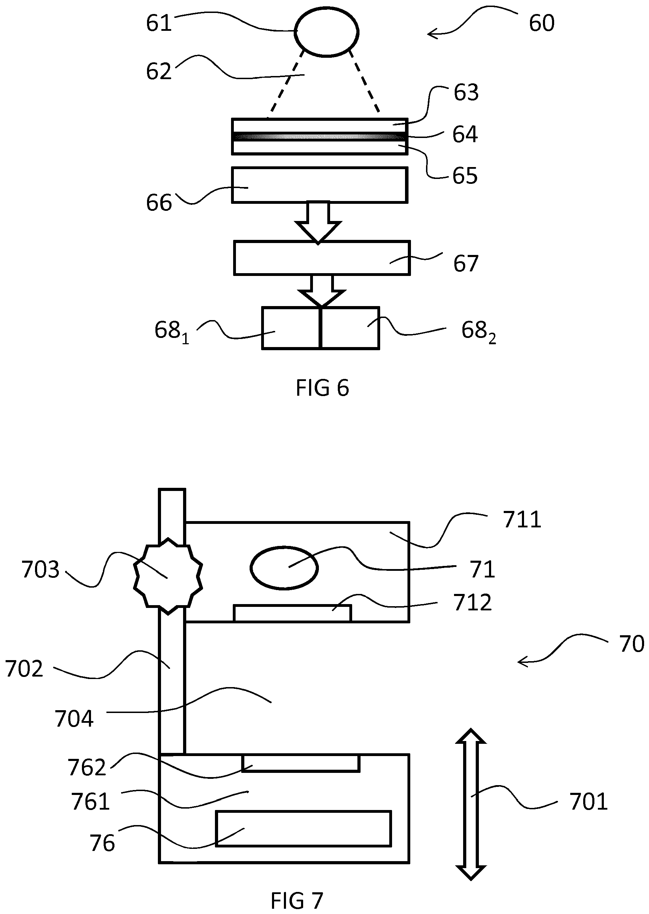

La Figure 6 illustre de façon schématique un dispositif 60 pour mettre en oeuvre le procédé selon l'invention. Sur la Figure 6, les références numériques 61, 62, 63, 64, 65, 66 correspondent respectivement aux références numériques 11, 12, 13, 14, 15 et 16 de la Figure 1.

La source lumineuse 61 est agencée pour illuminer un échantillon d'une solution liquide comprenant des cellules.

Le photodétecteur 66 est un photodétecteur matriciel disposé en face de la source lumineuse, et agencé pour acquérir une figure de diffraction globale de l'échantillon, la figure de diffraction globale comprenant plusieurs figures de diffraction élémentaires associées chacune à une cellule.

Le photodétecteur 66 est relié à des moyens de calcul 67 recevant en entrée la figure de diffraction globale telle que définie ci-avant.

Les moyens de calcul déterminent l'intensité lumineuse sur une surface centrale d'une figure de diffraction élémentaire associée à une cellule, et fournissent en sortie un classement de ladite cellule dans l'une parmi les deux catégories cellule vivante $68_1$ ou cellule morte $68_2$. La surface centrale est définie précédemment, pour illustrer le procédé selon l'invention.

En particulier, les moyens de calcul sont agencés pour :

- calculer l'indicateur numérique tel que défini précédemment ;
- comparer cet indicateur numérique à une valeur de seuil prédéterminée ;
- attribuer la cellule correspondant à ladite figure de diffraction élémentaire, à l'une parmi les deux catégories $68_1$ et $68_2$.

Les moyens de calcul comprennent notamment des moyens électroniques et des moyens informatiques et/ou logiciels. Il s'agit typiquement d'un circuit électronique numérique ou analogique, de préférence dédié, associé à un microprocesseur et/ou un ordinateur.

Le procédé selon l'invention peut avantageusement être mis en oeuvre pour contrôler *in situ* un traitement cellulaire dans un bioréacteur.

Le procédé selon l'invention utilise des figures de diffraction élémentaires associées chacune à une cellule. Pour que chaque figure de diffraction élémentaire soit exploitable, il faut que les figures ne soient pas superposées les unes aux autres. Pour une épaisseur donnée, l'échantillon 14 doit donc présenter une concentration en cellules inférieure à une concentration maximale prédéterminée.

Par exemple, dans le mode de réalisation décrit en référence à la Figure 1, on ne peut imager que jusqu'à 10000 figures de diffraction élémentaires sur le photodétecteur 16 présentant une surface de 24 mm$^2$. Cela correspond à une concentration maximale de 2*10$^6$ cellules/mL, le volume de l'échantillon 14 étant approximativement de 5 µL.

On voit donc qu'il peut être nécessaire de pouvoir adapter à la concentration en cellules de l'échantillon 14, un dispositif d'imagerie sans lentille utilisé pour mettre en oeuvre le procédé selon l'invention.

On propose à cette fin une sonde immergeable, destinée notamment à la mise en oeuvre du procédé selon l'invention. On pourra également envisager d'utiliser cette sonde immergeable pour d'autres utilisations. Pour cette raison, on a choisi dans la suite de décrire la sonde immergeable seule, indépendamment de moyens de calcul pour mettre en oeuvre des étapes du procédé selon l'invention.

Le principe mis en oeuvre dans cette sonde immergeable consiste à adapter l'épaisseur de l'échantillon à sa concentration en particules, ici des cellules.

La sonde immergeable est adaptée à des mesures réalisées en plongeant la sonde dans une solution liquide. On s'affranchit ainsi d'une étape de prélèvement d'échantillon et dépôt sur une lamelle. On limite également les perturbations de l'échantillon dues à son observation, puisque l'échantillon n'est pas isolé du reste de la solution liquide.

La Figure 7 illustre de façon schématique un premier mode de réalisation de sonde immergeable 70 pour mettre en oeuvre des étapes du procédé selon l'invention.

Les références 71 et 76 de la Figure 7 correspondent respectivement aux références 11 et 16 de la Figure 1. La source lumineuse 71 et le photodétecteur 76 forment donc ensemble un dispositif d'imagerie sans lentille tel que décrit en référence à la Figure 1.

La sonde immergeable 70 comprend un premier logement étanche 711, recevant la source lumineuse 71. La source lumineuse 71 est donc protégée à l'intérieur d'un logement étanche, ce qui permet d'immerger entièrement la source, sans risquer de l'endommager. Le premier logement étanche 711 présente une première fenêtre 712, transparente à la longueur d'onde d'émission de la source lumineuse 71. La première fenêtre 712 est située entre la source lumineuse 71 et le photodétecteur 76.

La sonde immergeable 70 comprend également un second logement étanche 761, recevant le photodétecteur 76. Le photodétecteur 76 est donc protégé à l'intérieur d'un logement étanche, ce qui permet de l'immerger entièrement sans risquer de l'endommager. Il ne s'agit pas simplement ici de recouvrir le photodétecteur d'une lamelle de protection, mais de l'envelopper entièrement à l'intérieur d'un boîtier. Le second logement étanche 761 présente une seconde fenêtre 762, transparente à la longueur d'onde d'émission de la source lumineuse 71. La seconde fenêtre 762 est située entre la première fenêtre 712 et le photodétecteur 76.

L'échantillon est formé par une portion de la solution liquide dans laquelle la sonde immergeable 70 est entièrement plongée, cette portion de solution liquide occupant l'espace 704 entre la première fenêtre transparente 712 et la seconde fenêtre transparente 762. La solution liquide comprend des particules telles que des cellules.

Le photodétecteur 76 est agencé pour acquérir une figure de diffraction globale comprenant une pluralité de figures de diffraction élémentaires, chacune associée à une particule. On souhaite adapter l'épaisseur de l'échantillon à sa concentration en cellules, de sorte qu'au moins 80% des figures de diffraction élémentaires d'une figure de diffraction globale ne soient pas superposées avec une autre figure de diffraction élémentaire. Pour cela, la distance entre la première fenêtre transparente 712 et la seconde fenêtre transparente 762 est variable.

Dans le mode de réalisation représenté sur la Figure 7, cette distance est variable dans le temps.

La première fenêtre transparente 712 est parallèle à la seconde fenêtre transparente 762. La seconde fenêtre transparente 762 est mobile selon un axe 701 orthogonal au plan commun des première et seconde fenêtres.

Le second logement 761 est fixe relativement à une tige 702 orientée selon l'axe 701. La tige 702 est crantée. Elle forme une crémaillère, entraînée en translation par un pignon (non représenté). Le pignon est mis en rotation par l'intermédiaire d'une molette 703. La tige 702, entraînant avec elle le second logement 761, peut donc être translatée selon l'axe 701 relativement au premier logement 711.

L'homme du métier pourra prévoir différentes variantes permettant de réaliser un déplacement d'une fenêtre transparente relativement à l'autre fenêtre transparente, selon un axe orthogonal au plan de ces deux fenêtres, sans sortir du cadre de la présente invention.

On peut ainsi ajuster la distance entre la première fenêtre transparente 712 et la seconde fenêtre transparente 762, en déplaçant la seconde fenêtre transparente 762 relativement à la première fenêtre transparente 712.

La sonde immergeable peut être reliée à des moyens de calcul (non représentés) tels que décrits ci-avant en référence à la Figure 6. En d'autres termes, le dispositif 60 tel que représenté à la Figure 6 peut comprendre une sonde immergeable pour mettre en oeuvre des étapes du procédé selon l'invention, la source lumineuse de la source immergeable formant la source lumineuse 61 et le photodétecteur de la sonde immergeable formant le photodétecteur 66.

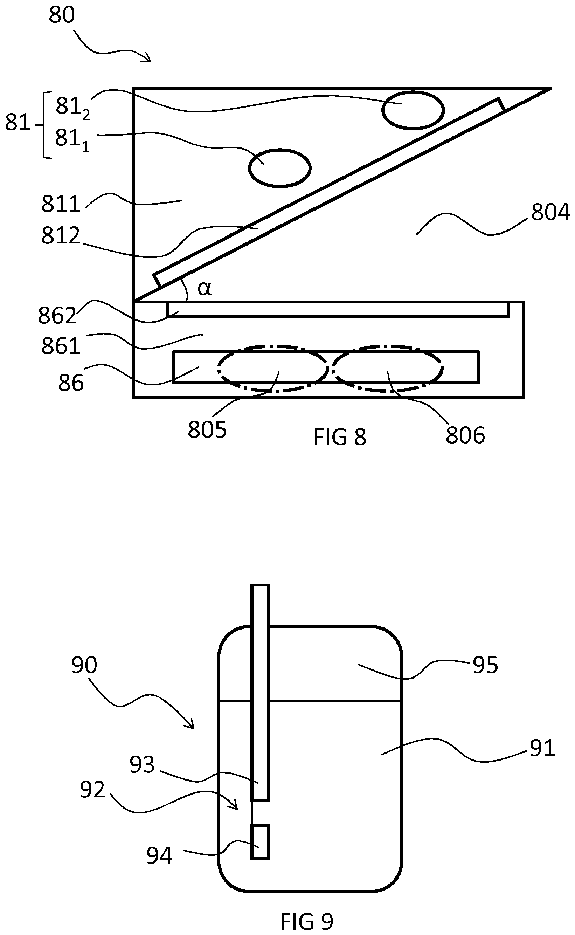

La Figure 8 illustre de façon schématique un deuxième mode de réalisation de sonde immergeable 80 pour mettre en oeuvre des étapes du procédé selon l'invention.

La sonde immergeable 80 ne sera décrite que pour ses différences relativement à la sonde immergeable 70. Sur la Figure 8, les références numériques 81, 811, 812, 86, 861, 862 et 804 correspondent respectivement aux références numériques 71, 711, 712, 76, 761, 762 et 704 de la Figure 7.

Dans le mode de réalisation représenté sur la Figure 8, la distance entre la première fenêtre transparente 812 et la seconde fenêtre transparente 862 est variable dans l'espace.

La première fenêtre transparente 812 est inclinée d'un angle $\alpha$ relativement à la seconde fenêtre transparente 862, ces deux fenêtres étant planes. L'angle $\alpha$ est typiquement compris entre 10° et 45°.

En variante, on pourrait prévoir une fenêtre plane, et une fenêtre présentant un profil en marches d'escalier.

La source lumineuse 81 est formée par plusieurs sources lumineuses unitaires $81_1$, $81_2$ réparties au-dessus de la première fenêtre transparente 812. Cette caractéristique n'est pas fondamentale, et on pourra également prévoir d'utiliser une source lumineuse faite d'une unique LED ou diode laser, comme décrit en référence à la Figure 1.

Une surface 805 du photodétecteur 86 correspond à une faible distance entre la première fenêtre transparente 812 et la seconde fenêtre transparente 862. On utilise cette surface 805 pour acquérir une figure de diffraction globale lorsque la solution liquide présente par exemple une forte concentration, par exemple supérieure à $10^6$ cellules/mL. La surface 805 est située en face de la source lumineuse unitaire $81_1$.

Une surface 806 du photodétecteur 86 correspond à une grande distance entre la première fenêtre transparente 812 et la seconde fenêtre transparente 862. On utilise cette surface 806 pour acquérir une figure de diffraction globale lorsque la solution liquide présente une faible concentration, par exemple comprise entre $10^4$ et $10^5$ cellules/mL. La surface 806 est située en face de la source lumineuse unitaire $81_2$.

On peut ainsi ajuster la distance entre la première fenêtre transparente 812 et la seconde fenêtre transparente 862, en définissant une portion du photodétecteur 86 utilisée pour obtenir des figures de diffraction élémentaires à analyser.

La Figure 9 illustre de façon schématique un bioréacteur 90 pour mettre en oeuvre des étapes du procédé selon l'invention. Le bioréacteur 90 désigne ici un dispositif destiné à cultiver des cellules ou des tissus dans le cadre d'une culture cellulaire, ou à mettre en oeuvre un processus chimique impliquant des organismes ou des substances biochimiques actives dérivées de ces organismes.

Un bioréacteur est conçu pour offrir des conditions optimales à une culture cellulaire ou un processus chimique. Pour cela, on ajuste et contrôle différents paramètres tels que :

- l'agitation de la solution liquide contenant les cellules, organismes ou substances biochimiques actives ;
- le pH de cette solution ;
- la température de cette solution ;
- le taux d'oxygène dissout dans cette solution.

Dans l'exemple représenté sur la Figure 9, le bioréacteur 90 reçoit une solution liquide 91 comprenant des cellules. Cette solution est située dans une cuve 95 du bioréacteur.

Des ouvertures pratiquées dans la cuve du bioréacteur permettent d'insérer des sondes pour contrôler les conditions à l'intérieur du bioréacteur 90. L'une de ces ouvertures est utilisée pour insérer une sonde immergeable 92 pour mettre en oeuvre des étapes du procédé selon l'invention. La sonde immergeable 92 présente une forme de cylindre allongé, de diamètre inférieur à 12 mm et de longueur supérieure à 50 mm. La sonde immergeable 92 présente une partie supérieure 93 recevant le premier logement étanche tel que décrit ci-avant, et une partie inférieure 94 recevant le second logement étanche tel que décrit ci-avant. L'espace entre ces deux logements est occupé par la solution liquide 91.

On peut ainsi mesurer l'évolution au cours du temps d'un taux de cellules vivantes dans le bioréacteur. Le temps d'acquisition d'une figure de diffraction globale est avantageusement de l'ordre de la milliseconde, pour éviter l'effet du mouvement des cellules.

## Revendications

1. Procédé pour discriminer une cellule vivante d'une cellule morte mis en oeuvre à l'aide d'un dispositif d'imagerie sans lentille (10) comprenant une source lumineuse (11; 61 ; 71 ; 81) et un photodétecteur matriciel (16 ; 66 ; 76 ; 86), dans lequel :

   - on illumine, à l'aide de la source lumineuse (11 ; 61 ; 71 ; 81), un échantillon (14 ; 64) d'une solution liquide comprenant des cellules; et
   - on acquiert, à l'aide du photodétecteur matriciel (16 ; 66 ; 76 ; 86) disposé en face de la source lumineuse, une figure de diffraction globale de l'échantillon, la figure de diffraction globale comprenant une pluralité de figures de diffraction élémentaires (20 ; 30) associées chacune à une cellule, chaque figure de diffraction élémentaire présentant une succession de courbes fermées concentriques ;

   **caractérisé en ce qu'**on classe une cellule dans l'une parmi les deux catégories cellule vivante ($68_1$) ou cellule morte ($68_2$), en fonction d'une intensité lumineuse sur une surface centrale (45 ; 46) d'une figure de diffraction élémentaire (20 ; 30) associée à ladite cellule.

2. Procédé selon la revendication 1, **caractérisé en ce**

**que** ladite surface centrale (45 ; 46) est inscrite dans une surface (41 ; 42) délimitée par un plus petit anneau de diffraction de la figure de diffraction élémentaire (20 ; 30).

3. Procédé selon la revendication 1 ou 2, **caractérisé par** les étapes suivantes :

   - on détermine une valeur d'un indicateur numérique représentatif de l'intensité lumineuse dans ladite surface centrale (45 ; 46) ;
   - on compare la valeur de l'indicateur numérique à une valeur de seuil prédéterminée ; et
   - en fonction du résultat de cette comparaison, on classe ladite cellule dans l'une parmi les deux catégories cellule vivante ($68_1$) ou cellule morte ($68_2$).

4. Procédé selon la revendication 3, **caractérisé en ce que** l'indicateur numérique est une intensité maximale ou une intensité moyenne ou une valeur crête-à-crête d'intensité.

5. Procédé selon la revendication 4, **caractérisé en ce que** la valeur de seuil prédéterminée est une intensité de référence, et **en ce que** :

   - une valeur de l'indicateur numérique inférieure à ladite intensité de référence correspond à une cellule vivante ; et
   - une valeur de l'indicateur numérique supérieure à ladite intensité de référence correspond à une cellule morte.

6. Procédé selon l'une quelconque des revendications 1 à 5, **caractérisé en ce qu'**il est mis en oeuvre pour effectuer un suivi *in situ* d'un traitement cellulaire dans un bioréacteur (90).

7. Procédé selon l'une quelconque des revendications 1 à 6, **caractérisé en ce que** l'on acquiert la figure de diffraction globale de l'échantillon, à l'aide d'un photodétecteur matriciel (16 ; 66 ; 76 ; 86) disposé en face de la source lumineuse, aucun dispositif de grossissement n'étant disposé entre l'échantillon (14; 64) et ledit photodétecteur matriciel.

8. Dispositif (60) pour discriminer une cellule vivante d'une cellule morte comprenant :

   - une source lumineuse (61), agencée pour illuminer un échantillon (64) d'une solution liquide comprenant des cellules ; et
   - un photodétecteur matriciel (66) disposé en face de la source lumineuse, formant avec la source lumineuse (61) un dispositif d'imagerie sans lentille, et agencé pour acquérir une figure de diffraction globale de l'échantillon, la figure de diffraction globale comprenant plusieurs figures de diffraction élémentaires (20 ; 30) associées chacune à une cellule, chaque figure de diffraction élémentaire présentant une succession de courbes fermées concentriques ;

   **caractérisé par** des moyens de calcul (67) :

   - recevant en entrée la figure de diffraction globale ;
   - déterminant l'intensité lumineuse sur une surface centrale (45 ; 46) d'une figure de diffraction élémentaire (20 ; 30) associée à une cellule ; et
   - fournissant en sortie un classement de ladite cellule dans l'une parmi les deux catégories cellule vivante ($68_1$) ou cellule morte ($68_2$).

9. Dispositif (60) selon la revendication 8, **caractérisé en ce qu'**aucun dispositif de grossissement n'est disposé entre l'échantillon (64) et le photodétecteur matriciel (66).

10. Dispositif (60) selon la revendication 8 ou 9, **caractérisé en ce qu'**il comprend :

    - un premier logement étanche (711 ; 811), recevant la source lumineuse (71 ; 81) et présentant une première fenêtre transparente (712 ; 812) entre la source lumineuse (711 ; 811) et le photodétecteur matriciel (76 ; 86) ;
    - un second logement étanche (761 ; 861), recevant le photodétecteur matriciel (76 ; 86), et présentant une seconde fenêtre transparente (762 ; 862) entre la première fenêtre transparente (712 ; 812) et le photodétecteur matriciel (76 ; 86) ;

    et **en ce que** la distance entre la première fenêtre transparente (712 ; 812) et la seconde fenêtre transparente (762 ; 862) est variable, le premier logement étanche (711 ; 811), la source lumineuse (71 ; 81), le second logement étanche (761 ; 861), et le photodétecteur matriciel (76 ; 86) formant ensemble une sonde immergeable (80 ; 70 ; 92).

11. Dispositif (60) selon la revendication 10, **caractérisé en ce que** la distance entre la première fenêtre transparente (712) et la seconde fenêtre transparente (762) est variable dans le temps, la première fenêtre transparente (712) et la deuxième fenêtre transparente (762) définissant deux plans parallèles entre eux, et le dispositif (60) comprenant des moyens pour déplacer la seconde fenêtre transparente relativement à la première fenêtre transparente.

12. Dispositif (60) selon la revendication 11, **caractérisé en ce que** les moyens pour déplacer la seconde fenêtre transparente (762) relativement à la première fenêtre transparente (712) comprennent une crémaillère (702) entraînée en translation par un pignon, et fixe relativement au second logement étanche (761).

13. Dispositif (60) selon la revendication 10, **caractérisé en ce que** la distance entre la première fenêtre transparente (812) et la seconde fenêtre transparente (862) est variable dans l'espace.

14. Dispositif (60) selon la revendication 13, **caractérisé en ce que** la première fenêtre transparente (812) et la deuxième fenêtre transparente (862) définissent deux plans inclinés l'un relativement à l'autre, de sorte que la distance entre la première fenêtre transparente (812) et la seconde fenêtre transparente (862) dépend de l'emplacement sur la seconde fenêtre transparente.

15. Dispositif (60) selon l'une quelconque des revendications 8 à 14, **caractérisé en ce que** les moyens de calcul déterminent l'intensité lumineuse sur une surface centrale (45 ; 46) d'une figure de diffraction élémentaire (20 ; 30) associée à une cellule, la surface centrale (45 ; 46) étant inscrite dans une surface (41 ; 42) délimitée par un plus petit anneau de diffraction de la figure de diffraction élémentaire (20 ; 30).

**Patentansprüche**

1. Verfahren zur Unterscheidung einer lebenden Zelle von einer toten Zelle, ausgeführt mit Hilfe einer ohne Linse Abbildungsvorrichtung (10), umfassend eine Lichtquelle (11 ; 61 ; 71 ; 81) und einen matrixförmigen Fotodetektor (16 ; 66 ; 76 ; 86), bei dem ;

     - eine Probe (14 ; 64) einer Zellen enthaltenden flüssigen Lösung mit Hilfe der Lichtquelle (11 ; 61 ; 71 ; 81) beleuchtet wird; und
     - eine Globaldiffraktionsfigur der Probe mit Hilfe des der Lichtquelle gegenüberliegend angeordneten matrixförmigen Fotodetektors (16 ; 66 ; 76 ; 86) erfasst wird, wobei die Globaldiffraktionsfigur eine Vielzahl Elementardiffraktionsfiguren (20 ; 30) umfasst, von denen jede mit einer Zelle verbunden ist, wobei jede Elementardiffraktionsfigur eine Folge von konzentrischen geschlossenen Kurven aufweist;

     **dadurch gekennzeichnet, dass** eine Zelle in Abhängigkeit von einer Lichtstärke in einer zentralen Fläche (45 ; 46) einer mit der genannten Zelle verbundenen Elementardiffraktionsfigur (20 ; 30) in eine der beiden Kategorien *Lebende Zelle* ($68_1$) oder *Tote Zelle* ($68_2$) einsortiert wird.

2. Verfahren nach Anspruch 1, **dadurch gekennzeichnet, dass** die genannte zentrale Fläche (45 ; 46) eingeschrieben ist in einer Fläche (41 ; 42), begrenzt durch einen kleineren Diffraktionsring der Elementardiffraktionsfigur (20 ; 30).

3. Verfahren nach Anspruch 1 oder 2, **gekennzeichnet durch** die folgenden Schritte :

     - ein Wert eines für die Lichtstärke in der genannten zentralen Fläche (45 ; 46) repräsentativen numerischen Indikators bestimmt wird ;
     - der Wert des numerischen Indikators mit einem Schwellenwert verglichen wird; und
     - die genannte Zelle in eine der beiden Kategorien *Lebende Zelle* ($68_1$) oder *Tote Zelle* ($68_2$) in Abhängigkeit von dem Ergebnis dieses Vergleichs einsortiert wird.

4. Verfahren nach Anspruch 3, **dadurch gekennzeichnet, dass** der numerische Indikator eine maximale Stärke oder eine mittlere Stärke oder ein Spitze-Spitze-Stärkewert ist.

5. Verfahren nach Anspruch 4, **dadurch gekennzeichnet, dass** der bestimmte Schwellenwert eine Referenzstärke ist, und dadurch, dass ;

     - ein numerischer Indikatorwert, der kleiner als die genannte Referenzstärke ist, einer lebenden Zelle entspricht; und
     - ein numerischer Indikatorwert, der größer als die genannte Referenzstärke ist, einer toten Zelle entspricht.

6. Verfahren nach einem der Ansprüche 1 bis 5, **dadurch gekennzeichnet, dass** es ausgeführt wird, um eine *in situ*-Überwachung einer Zellenbehandlung in einem Bioreaktor (90) vorzunehmen.

7. Verfahren nach einem der Ansprüche 1 bis 6, **dadurch gekennzeichnet, dass** die Globaldiffraktionsfigur der Probe mit Hilfe eines der Lichtquelle gegenüberliegend angeordneten Fotodetektors (16 ; 66 ; 76 ; 86) erlangt wird, wobei zwischen der Probe (14 ; 64) und dem genannten matrixförmigen Fotodetektor keine Vergrößerungseinrichtung angeordnet ist.

8. Vorrichtung zur Unterscheidung einer lebenden Zelle von einer toten Zelle, umfassend :

     - eine Lichtquelle (61), eingerichtet um eine Probe (64) einer Zellen enthaltenden flüssigen Lösung zu beleuchten ; und
     - einen matrixförmigen Fotodetektor (66), der Lichtquelle gegenüberliegend angeordnet, mit der Lichtquelle (61) eine ohne Linse Abbildungsvorrichtung bildend und eingerichtet, um eine Globaldiffraktionsfigur der Probe zu erlangen, wobei die Globaldiffraktionsfigur mehrere Elementardiffraktionfiguren (20 ; 30) umfasst, von denen jede mit einer Zelle verbunden ist, wobei jede Elementardiffraktionsfigur eine Folge von konzentrischen geschlossenen Kurven aufweist;

**gekennzeichnet durch** Recheneinrichtungen (67):

- die als Eingang die Globaldiffraktionfigur erhalten ;
- die die Lichtstärke in einer zentralen Fläche (45 ; 46) einer einer Zelle zugeordneten Elementardiffraktionsfigur (20 ; 30) bestimmen ; und
- die als Ausgang eine Einsortierung der genannten Zelle in eine der beiden Kategorien *Lebende Zelle* ($68_1$) oder *Tote Zelle* ($68_2$) liefern.

9. Vorrichtung (60) nach Anspruch 8, **dadurch gekennzeichnet, dass** zwischen der Probe (64) und dem matrixförmigen Fotodetektor (66) keine Vergrößerungseinrichtung angeordnet ist.

10. Vorrichtung (60) nach Anspruch 8 oder 9, **dadurch gekennzeichnet, dass** sie umfasst:

- ein erstes dichtes Gehäuse (711 ; 811) zur Aufnahme der Lichtquelle (71 ; 81), der auch ein erstes transparentes Fenster (712 ; 812) zwischen der Lichtquelle (711 ; 811) und dem matrixförmigen Fotodetektor (76 ; 86) aufweist;
- ein zweites dichtes Gehäuse (761 ; 861) zur Aufnahme des matrixförmigen Fotodetektors (76 ; 86), der auch ein zweites transparentes Fenster (762 ; 862) zwischen dem ersten transparenten Fenster (712 ; 812) und dem matrixförmigen Fotodetektors (76 ; 86) aufweist;

und dadurch, dass der Abstand zwischen dem ersten transparenten Fenster (712 ; 812) und dem zweiten transparenten Fenster (762 ; 862) variabel ist, wobei das erste dichte Gehäuse (711 ; 811), die Lichtquelle (71 ; 81), das zweite dichte Gehäuse (761 ; 861), und der matrizenförmige Fotodetektor (76 ; 86) zusammen eine untertauchbare Tauchsonde (80 ; 70 ; 92) bilden.

11. Vorrichtung (60) nach Anspruch 10, **dadurch gekennzeichnet, dass** der Abstand zwischen dem ersten transparenten Fenster (712) und dem zweiten transparenten Fenster (762) zeitlich variabel ist, wobei das erste transparente Fenster (712) und das zweite transparente Fenster (762) zwei zueinander parallele Ebenen definieren, und die Vorrichtung (60) Einrichtungen zum Verschieben des zweiten transparenten Fensters in Bezug auf das erste transparente Fenster umfasst.

12. Vorrichtung (60) nach Anspruch 11, **dadurch gekennzeichnet, dass** die Einrichtungen zum Verschieben des zweiten transparenten Fensters (762) in Bezug auf das erste transparente Fenster (712) eine Zahnstange (702) umfassen, translatorisch angetrieben durch ein Ritzel und ortsfest in Bezug auf das zweite dichte Gehäuse (761).

13. Vorrichtung (60) nach Anspruch 10, **dadurch gekennzeichnet, dass** der Abstand zwischen dem ersten transparenten Fenster (812) und dem zweiten transparenten Fenster (862) räumlich variabel ist.

14. Vorrichtung (60) nach Anspruch 13, **dadurch gekennzeichnet, dass** das erste transparente Fenster (812) und das zweite transparente Fenster (862) zwei zueinander geneigte Ebenen definieren, so dass der Abstand zwischen dem ersten transparenten Fenster (812) und dem zweiten transparenten Fenster (862) von dem Platz über dem zweiten transparenten Fenster abhängt.

15. Vorrichtung (60) nach einem der Ansprüche 8 bis 14, **dadurch gekennzeichnet, dass** die Recheneinrichtungen die Lichtstärke in einer zentralen Fläche (45 ; 46) einer einer Zelle zugeordneten Elementardiffraktionsfigur (20 ; 30) bestimmen, wobei die zentrale Fläche (45 ; 46) eingeschrieben ist in einer Fläche (41 ; 42), begrenzt durch einen kleineren Diffraktionsring der Elementardiffraktionsfigur (20 ; 30).

**Claims**

1. Method of discriminating a living cell from a dead cell implemented using a lens-free imaging device (10) comprising a light source (11; 61; 71; 81) and a matrix photodetector (16; 66; 76; 86), in which:

- the light source (11; 61; 71; 81), is used to illuminate a sample (14; 64) of a liquid solution containing cells; and
- the matrix photodetector (16; 66; 76; 86) is arranged facing the light source, and acquires a global diffraction figure of the sample, the global diffraction figure comprising a plurality of elementary diffraction figures (20; 30) each associated with a cell, each elementary diffraction figure having a succession of closed concentric curves;

**characterised in that** a cell is classified in one among the living cells category ($68_1$) or the dead cells category ($68_2$), depending on a light intensity on a central area (45; 46) of an elementary diffraction figure (20; 30) associated with said cell.

2. Method according to claim 1, **characterised in that** said central area (45; 46) is inscribed in an area (41; 42) delimited by a smallest diffraction ring of the elementary diffraction figure (20; 30).

3. Method according to claim 1 or 2, **characterised by** the following steps:

   - a value of a numeric indicator representative of the light intensity in said central area (45; 46) is determined;
   - the value of the numeric indicator is compared with a predetermined threshold value; and
   - depending on the result of this comparison, said cell is classified in one among the living cells category (68$_1$) or the dead cells category (68$_2$).

4. Method according to claim 3, **characterised in that** the numeric indicator is a maximum intensity or a mean intensity or a peak-to-peak intensity value.

5. Method according to claim 4, **characterised in that** the predetermined threshold value is preferably a reference intensity, and **in that**:

   - a numeric indicator value lower than said reference intensity corresponds to a living cell; and
   - a numeric indicator value higher than said reference intensity corresponds to a dead cell.

6. Method according to any one of claims 1 to 5, **characterised in that** it is used to perform *in situ* monitoring of a cell treatment in a bioreactor (90).

7. Method according to any one of claims 1 to 6, **characterised in that** the global diffraction figure of the sample is acquired using a matrix photodetector (16; 66; 76; 86) placed facing the light source, no magnification device being placed between the sample (14; 64) and said matrix photodetector.

8. Device (60) for discriminating a living cell from a dead cell comprising:

   - a light source (61), arranged to illuminate a sample (64) of a liquid solution comprising cells; and
   - a matrix photodetector (66) placed facing the light source, forming a lens-free imaging device with the light source (61), and arranged to acquire a global diffraction figure of the sample, the global diffraction figure comprising several elementary diffraction figures (20; 30) each of which is associated with a cell, each elementary diffraction figure having a succession of closed concentric curves;

   **characterised by** calculation means (67):

   - receiving the global diffraction figure as input;
   - determining the light intensity on a central area (45; 46) of an elementary diffraction figure (20; 30) associated with a cell; and
   - providing as output a classification of said cell as a living cell (68$_1$) or dead cell (68$_2$).

9. Device (60) according to claim 8, **characterised in that** no magnification device is placed between the sample (64) and the matrix photodetector (66).

10. Device (60) according to claim 8 or 9, **characterised in that** it comprises:

    - a first sealed housing (711; 811), holding the light source (71; 81) and having a first transparent window (712; 812) between the light source (711; 811) and the matrix photodetector (76; 86);
    - a second sealed housing (761; 861), holding the matrix photodetector (76; 86), and having a second transparent window (762; 862) between the first transparent window (712; 812) and the matrix photodetector (76; 86);

    and **in that** the distance between the first transparent window (712; 812) and the second transparent window (762; 862) is variable, the first sealed housing (711; 811), the light source (71; 81), the second sealed housing (761; 861), and the matrix photodetector (76; 86) together forming an immersible probe (80; 70; 92).

11. Device (60) according to claim 10, **characterised in that** the distance between the first transparent window (712) and the second transparent window (762) is variable in time, the first transparent window (712) and the second transparent window (762) defining two planes parallel to each other, and the device (60) comprising means of displacing the second transparent window relative to the first transparent window.

12. Device (60) according to claim 11, **characterised in that** the means for displacing the second transparent window (762) relative to the first transparent window (712) include a rack (702) driven in translation by a gear, and fixed relative to the second sealed housing (761).

13. Device (60) according to claim 10, **characterised in that** the distance between the first transparent window (812) and the second transparent window (862) is variable in space.

**14.** Device (60) according to claim 13, **characterised in that** the first transparent window (812) and the second transparent window (862) define two planes inclined relative to each other, such that the distance between the first transparent window (812) and the second transparent window (862) depends on the location on the second transparent window.

**15.** Device (60) according to any one of claims 8 to 14, **characterised in that** the calculation means determine the light intensity on a central area (45; 46) of an elementary diffraction figure (20; 30) associated with a cell, the central area (45; 46) being inscribed in an area (41; 42) delimited by a smallest diffraction ring in the elementary diffraction figure (20; 30).

FIG 1

FIG 2A

FIG 2B

FIG 3A

FIG 3B

FIG 4A

45

41

FIG 4B

42

46

FIG 4C

45

451

FIG 4D

46

461

FIG 5

61
62

60

63
64
65
66

67

68₁ 68₂

FIG 6

711
703
71
712
702
70
704

762
761
76
701

FIG 7

FIG 8

FIG 9

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 6905838 B

- WO 2012062805 A

**Littérature non-brevet citée dans la description**

- **TING-WEI SU.** High-throughput lensfree imaging and characterization of a heterogeneous cell solution on a chip. *Biotechnology and bioengineering,* 15 Février 2009, vol. 102 (3